## Europäisches Patentamt

## European Patent Office

## Office européen des brevets

(11) Publication number: **0 157 253**
**A1**

(12) **EUROPEAN PATENT APPLICATION**

(21) Application number: **85102962.9**

(22) Date of filing: **14.03.85**

(51) Int. Cl.⁴: **C 12 N 15/00**

(30) Priority: **05.04.84 US 597138**

(43) Date of publication of application: **09.10.85**
**Bulletin 85/41**

(84) Designated Contracting States: **BE DE FR GB IT LU NL SE**

(71) Applicant: **Cyanotech Corporation, 18748 - 142nd N.E., Woodinville, Washington 98072 (US)**

(72) Inventor: **Karuna-Karan, Arthur, 15848-32nd Avenue N. E., Seattle Washington 98155 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath, Maximilianstrasse 58, D-8000 München 22 (DE)**

(54) **Comspositions and methods for dna cloning in cyanobacteria.**

(57) A novel vehicle for cloning DNA in cyanobacteria and other Gram-negative bacteria includes a conjugative replication system and a replication system derived from a suitable cyanobacterial plasmid. The cloning vehicles have enhanced replication function and are able to be introduced into cyanobacterial hosts from other convenient cloning hosts, such as E. coli. The cloning vehicles, while being non-selftransmissible, may be mobilized in the presence of a helper plasmid which provides the necessary transfer functions by trans-complementation. Exogenous DNA may be inserted into the cloning vehicle and conjugally transferred to a target host when the helper plasmid is present on the same or second donor host.

ACTORUM AG

COMPOSITIONS AND METHODS FOR DNA
CLONING IN CYANOBACTERIA

Background of the Invention

1.  Field of the Invention

The construction and characterization of recombinant DNA molecules in microorganisms depends on the availability of cloning vectors capable of being introduced and maintained in the microorganism host of interest.  At present, the majority of well-characterized cloning vectors have been constructed from plasmids and/or bacteriophages having a limited host range, typically being limited to enteric bacteria.

It is thus desirable to provide additional cloning vectors and cloning systems capable of efficient introduction into and stable maintenance within a wide variety of microorganisms.  Such cloning vectors and systems are preferably transfer deficient to prevent accidental introduction of genetic material into other microorganisms which may be present.  Moreover, the cloning vectors should include restriction sites suitable for incorporation of exogenous DNA and selectable markers which are expressed in each type of host, where at least some of the selectable markers allow for the screening of recombinants by insertional inactivation.  Also, suitable cloning vectors should be small in size, preferably below about 20kbp, to allow for insertion of relatively large DNA fragments with efficient transformation.

Even when suitable cloning vectors exist, it is frequently difficult to efficiently introduce the vectors to a particular host because of the very low transformation efficiencies associated with conventional techniques, such as calcium precipitation. It is thus desirable to provide highly efficient cloning systems, such as conjugal mating systems, for a wide variety of biologically important bacteria.

## 2.    Description of the Prior Art

Various cyanobacterial strains, including Anacystis nidulans, Gloecapsa alpicola, and Agmenellum quadruplicatum, have been transformed with bare DNA. See Shestakov and Khyen (1970) Molec. Gen. Genet. 107:372-375; Devilly and Houghton (1977) J. Gen. Microbiol. 98:277-280; and Stevens and Porter (1980) Proc. Natl. Acad. Sci. USA 77:6052-6056. Transformation of Anacystis nidulans with an endogenous plasmid (pUH24) carrying a transposon (Tn901) which imparts ampicillin resistance has been accomplished. van den Hondel et al. (1980) Proc. Natl. Acad. Sci. USA 77:1570-1574.  Shuttle vectors including origins of replication for both Anacystis nidulans and E. coli have been constructed.  Kuhlemeier et al., (1981) Mol. Gen. Genet. 184:249-254.

## Summary of the Invention

Novel cloning vehicles capable of efficiently introducing heterologous DNA into a broad range of cyanobacterial hosts are provided.  The cloning vehicles include a replication system and origin of transfer derived from a conjugative plasmid as well as at least one DNA sequence derived from cyanobacteria. The cyanobacterial DNA sequence may include a replication system providing for stable maintenance of the cloning vehicle in the cyanobacterial genome allowing for introduction by homologous recombination. The cloning vehicle will also include at least one restriction site for insertion of a DNA fragment to be cloned, and will usually include one or more selective markers allowing for selection of transformed hosts.

The cloning vehicles are transfer deficient, but can be mobilized for conjugal transfer by a suitable helper plasmid. Thus, transformation of desired recipient cells can be effected in the presence of the helper plasmid, while transfer of the cloning vehicle to other microorganisms is avoided in the absence of the helper plasmid. A wide variety of Gram-negative bacteria are suitable as the donor cells, while the recipient cells will be generally (although not necessarily) limited to cyanobacteria. The cloning vehicle can be introduced into the recipient cell either by transformation with the cloning vehicle and helper plasmid present in the same donor cell, or by tri-parental mating with the cloning vehicle and helper plasmid initially present in separate donor cells. In either case, efficient transformation not available with non-conjugal transfer systems can be achieved.

## Description of the Specific Embodiments

Methods and compositions are provided for the introduction and cloning of exogenous DNA into a wide variety of cyanobacteria and other Gram-negative hosts. Novel plasmid cloning vehicles are employed which include a replication system derived from a conjugative plasmid and a sequence of at least 500 base pairs derived from cyanobacterial DNA. The plasmid cloning vehicle will carry the origin of transfer for the conjugative mating system, but will not include the other necessary transfer functions. Thus, the plasmid vehicle will be non-self-transmissible, yet will be capable of broad host range transmissibility in combination with a helper plasmid carrying the necessary transfer functions. The cloning vehicle will include at least one restriction site in a non-essential region of the plasmid for the insertion of the exogenous DNA. Usually the cloning vehicle will have at least one marker allowing for selection, and preferably will include a selectable marker which is associated with a unique restriction site, allowing for selection of transformants by insertional inactivation of the marker.

The cyanobacterial DNA sequence may comprise either a replication system derived from a cyanobacterial plasmid or a fragment obtained from a cyanobacterial genome. When the cloning vehicle carries the cyanobacterial replication system, the vehicle will be stably maintained in all cyanobacterial strains which recognize the system. Replication and expression of the exogenous DNA will occur on the plasmid. When the cloning vehicle carries only genomic DNA, however, maintenance of the exogenous DNA in the recipient host occurs only as the result of homologous exchange between the plasmid and the host. By providing only a single homologous region in the plasmid, a single cross-over may occur which results in incorporation of the entire plasmid (including the exogenous DNA) into the host genome. By providing two homologous regions, a double cross-over may occur which results in limited transfer of the region which lies between the two homologous regions. Moreover, exogenous DNA inserted within a single homologous sequence on the cloning vehicle effectively creates a pair of homologous regions and allows a double cross-over event which can result in incorporation of the exogenous DNA on the homologous region in the cyanobacterial genome.

The cloning vehicle is capable of introduction and stable maintenance in a broad range of cyanobacteria and other Gram-negative bacteria. Suitable cyanobacteria hosts include <u>Anacystis</u>, such as <u>A. nidulans</u>; <u>Spirulina</u>, such as <u>S. platensis</u>, <u>S. maxima</u>, <u>S. major</u>, and <u>S. sub salsa versicala</u>; <u>Anabaena</u>, such as <u>A. flos-aquae</u>, and <u>A. cylindrica</u>; <u>Mastigocladus</u>, such as <u>M. laminosus</u>; and <u>Tolypothrix</u>, such as <u>T. tenuis</u>.

Suitable Gram-negative hosts include enterobacteri-
aceae, such as Escherichia, Klebsiella, Providentia,
Serratia, Erwinia; rhizobiaceae, such as R. rhizobium
and Agrobacterium; and pseudomonadaceae, such as
Pseudomonas.

Generally, only Gram-negative strains other
than cyanobacteria will be suitable as the donor,
particularly these strains which provide for efficient
conjugal transfer. Moreover, the usefulness of the
present invention is generally limited to
cyanobacterial recipients, although the cloning
vehicles can be transfered to many other Gram-negative
hosts as well.

Conveniently, the cloning vector can be
manipulated in a well-characterized cloning host, such
as E. coli, in order to construct a recombinant plasmid
carrying the DNA insert(s) of interest. After the
desired insertions and modifications have been made to
the cloning vehicle, the recombinant vehicle may be
introduced into the desired recipient host, usually a
cyanobacterial host, although the modified cloning
vehicle may be suitable for introduction into other
Gram-negative hosts as well.

The cloning vehicle of the present invention
is incapable of self-transmissibility. That is, it
lacks at least one gene function required for conjugal
transfer. Usually, a substantial portion of one or
more transfer genes encoding for the transfer
function(s) will be deleted. Not all of the transfer
genes need be deleted, however, and when the transfer
genes overlap other essential functions of the plasmid,
those regions will be retained. The gene transfer
function(s) which have been deleted or otherwise
rendered inactive on the cloning vehicle will be
provided on a second plasmid, referred to as a helper
plasmid,which provides the necessary transfer functions
by transcomplementation. In this way, the cloning vehicle can be transferred to a desired recipient.
bacteria in the presence of the helper plasmid, but
cannot be conjugally transferred to other bacterial
hosts when the helper plasmid is not present.

Conjugal transfer may be effected using
either one or two donor bacteria. When using one donor
bacterium, both the cloning vehicle and the helper
plasmid will be introduced to the same donor bacterium
using conventional transformation techniques, such as
calcium precipitation. Conveniently, the donor
bacterium will be $\underline{E}$. $\underline{coli}$ or other Gram-negative
well-characterized bacterium. Conjugal transfer is
then effected by mixing the donor bacterium together
with the recipient cyanobacterial strain in a suitable
selective medium for an extended period of time,
typically at least 6 hours, more typically 12 hours.
When the cloning plasmid and the helper plasmid are
initially present in the same donor, it is desirable to
use a recombination deficient ($\underline{RecA}^-$) bacterium to
prevent recombination between the plasmids which share
substantial homology.

The use of two donor strains is similar,
except that the cloning plasmid and helper plasmid are
transformed into separate strains of bacteria. The two
donor strains are then mixed with the recipient strain
in a suitable medium. Initially, the helper plasmid
will be conjugally transfered to the donor strain
carrying the cloning plasmid. The helper plasmid will
provide the necessary transfer factors absent from the
cloning vehicle, and the cloning vehicle will be
capable of conjugal transfer to the desired recipient.
Cyanobacterial transformants can be selected on a
minimal medium which will not support the growth of the
donor bacteria and which is further selective for a
marker present on the cloning vehicle.

A wide variety of markers may be employed.
Conveniently, antibiotic resistance allows for selection of transformants by culturing the recipient cells on a medium containing the particular antibiotic. Heavy metal resistance can be employed in an analogous manner. Alternatively, the cloning vehicle may carry the necessary genes to provide essential metabolites to an auxotrophic host. By culturing the transformation mixture in a medium lacking the essential metabolite, transformants can be selected. Other well known selection techniques include providing incompatibility with particular bacteriophage strains, resistance to toxins, changes in morphology, and the like. It is often desirable to provide more than one marker, particularly where one of the markers includes a restriction site for insertion of the exogenous DNA. Transformants may then be initially selected based on the presence of a first marker, while those transformants carrying the desired recombinant plasmids may be screened for loss of a second marker.

The cloning vehicle will normally include at least one unique restriction site and may include many more. The restriction sites are provided in nonessential regions of the cloning vehicles so that insertion of exogenous DNA will not disturb the normal functioning of the plasmid vehicle. When the cloning vehicle carries cyanobacterial genomic DNA, it is desirable that the homologous sequence include a unique restriction site for introduction of the exogenous DNA. The restriction site should be located so that the homologous region extends at least 500 base pairs in both directions, preferably at least 1000 base pairs in both directions.

The nature of the exogenous DNA which is to be introduced may vary widely. Virtually any DNA sequence may be cloned by introduction into a suitable restriction site on the plasmid vehicle, introduction of the recombinant plasmid into a suitable host, and cloning of the plasmid. When it is desired to obtain expression of an inserted gene, the necessary regulatory functions, including promoter, start and stop codons, terminator, and the like, will usually be introduced together with the structural gene. Alternatively, the cloning vector itself may carry the necessary regulatory sequences. In either case, regulatory systems must be recognized by the target host.

The cloning vehicle of the present invention may be prepared from existing conjugal cloning systems such as IncP systems, e.g., the pRK290/pRK2013 system described by Ditta et al. (1980) Proc. Natl. Acad. Sci. USA 77:7347-7351, or IncW systems, e.g., the pSa151/pSa322 system described by Tait et al. (1983) "Construction of Cloning Vectors From the IncW Plasmid pSa and their Use in Analysis of Crown Gall Tumor Formation" in: Genetic Engineering in Eukaryotes, Lurquin and Kleinhofs, eds., pp. 111-123, Plenum Publishing Company, New York, New York. The existing cloning vectors are modified by insertion of the cyanobacterial replication system at an available restriction site in a non-essential region of the vehicle. For example, pRK290 includes unique BglII and EcoRI restriction sites where the replication system can be inserted, and plasmid pSa151 includes unique PvuII and BamHI restriction sites which are suitable for insertion of the cyanobacterial replication system. Plasmid pVK102, a derivative of pRK290, is utilized in the Experimental section hereinafter.

The cyanobacterial replication system can be obtained from suitable cyanobacterial plasmids, such as pUH24 and pUH25 obtained from Anacystis nidulans strain R2. The cyanobacterial plasmids may be isolated by the method of van den Hondel et al. (1979) Plasmid 2:323-333. Plasmid pUH24, which is about 8.0kbp in length, can be cleaved with EcoRI and inserted directly into the EcoRI site of pRK290. It will usually be desirable, however, to reduce the length of the in-serted fragment, either by further restriction or by chewing back using exonuclease Bal31.

Homologous genomic DNA may be obtained by restricting or partially restricting the genome of the cyanobacterial strain of interest. Fragments in the desired size range may then be selected and incorporated into the plasmid by conventional techniques.

The following examples are offered by way of illustration, not by way of limitation.

EXAMPLES

A.    Preparation of recombinant vectors.

Two types of vectors suitable for transforming both E. coli and Spirulina are constructed. One vector permits autonomous replication in both E. coli and Spirulina. This vector is constructed by recombining an E. coli plasmid and a Spirulina plasmid. The second vector contains an E. coli plasmid and a fragment of the Spirulina chromosome. The Spirulina DNA fragment permits this vector to integrate into the Spirulina genome by homologous recombination.

1.    Autonomously replicating vector

Plasmid DNA is prepared from Spirulina by the procedure of Currier and Nester (1976) Anal. Biochem. 76:431-441 and purified by cesium chloride centrifugation. The plasmid DNA is then partially digested with the restriction endonuclease SalI (BRL, Inc.) such that the entire Spirulina plasmid is present as linear DNA fragments with SalI cohesive ends. The SalI fragments are mixed with the wide host range plasmid pVK102 (Knauf and Nestor (1982) Plasmid 8:45-54) that has been digested to completion with SalI. The plasmid pVK102 contains a single SalI site in the gene for resistance to tetracycline. The mixture is ligated with T4 ligase (BRL, Inc.) and transformed into E. coli strain mm294 (Maniatis et al. (1982) Molecular Cloning, Cold Springs Harbor Laboratory, p. 255). E. coli colonies are selected for resistance to kanamycin (50 µg/ml, Sigma) which is encoded by the neomycin phosphotransferase (NPTII) gene present on pVK102. The kanamycin resistant colonies are tested for sensitivity to tetracycline (10 µg/ml, Sigma). Tetracycline sensitivity indicates insertional inactivation of the tetracycline resistance gene at the SalI site of pVK102 by a SalI fragment of Spirulina plasmid DNA. The structures of the recombinant plasmids are analyzed by the rapid plasmid screening procedure of Birnboim and Doly (1979) Nucl. Acids Res. 7:1513-1523 to confirm the insertion of Spirulina plasmid into pVK102.

2.    Integrating vector

Chromosomal DNA is prepared from Spirulina by modification of the procedure of J. Marmur (1983) Methods in Enzymology 6:726-738. The DNA is digested to completion with the restriction endonuclease SalI. Single SalI fragments are ligated into SalI digested pVK102. The ligated DNA is transformed into E. coli, selected for resistance to kanamycin, and tested for tetracycline sensitivity. The clones are then screened by the Birnboim and Doly (supra.) procedure, and clones containing single SalI fragments of two kilobase pairs (kb) or greater in size are selected for use as integration vectors.

B.    Introduction of recombinant DNA into Spirulina

The autonomous replication and integration vectors are introduced into Spirulina from E. coli in the same manner.  An exponentially growing culture of Spirulina is subjected to vigorous agitation to knead up the filaments into single cells.  The cells are collected on a 0.45 micron filter disc.  Approximately $10^{\varepsilon}$ E. coli cells containing the recombinant vector and the trans acting conjugal plasmid pRK2073 (Ditta et al., supra) are added to the filter.  The filter is incubated at 30°C for 24 hours on a nutrient agar (Difco) plate.  Following the incubation, the cells are washed off the filter and grown for 24 hours in liquid Spirulina medium (Zarrouk (1966) Doctoral Thesis, University of Paris).  The incubation in the Spirulina media without antibiotics insures that the Spirulina has resumed exponential growth and has overcome any inhibition from the nutrient agar incubation that might affect expression of the NPTII gene in Spirulina.  It also allows for expression of the NPTII gene before the kanamycin substrate is present and reduces the E. coli population which cannot grow in the minimal Spirulina medium.  Transformed Spirulina cells are selected by plating the mating mixture on Spirulina agar plates containing 100 µg/ml of kanamycin.

C.    Assay for transfer and expression of NPTII in Spirulina

Kanamycin resistant Spirulina transformants are tested for the presence of the NPTII gene by two procedures.  Total (chromosomal and plasmid) DNA is prepared from cultures of kanamycin resistant Spirulina isolates and probed for the presence of the NPTII gene by DNA-DNA hybridization using the procedure of E. M. Southern (1975) J. Mol. Biol. 98:503-517.  Transformed isolates containing the NPTII gene are screened further for the presence of the gene product with antibody specific for the NPTII protein (A. Jiminez and J. Davis (1980) Nature 287:869-871).

Although the foregoing invention has been described in some detail by way of illustration and example for purposes of clarity of understanding, it will be obvious that certain changes and modifications may be practiced within the scope of the appended claims.

0157253

WHAT IS CLAIMED IS:

1. A plasmid cloning vehicle including at least one insertional site and having (1) a DNA sequence of at least 500 base pairs derived from a cyanobacterial plasmid and (2) a replication system and an origin of transfer derived from a conjugative plasmid, said plasmid cloning vehicle lacking self-transmissibility but being capable of conjugal transfer from Gram-negative bacteria to cyanobacteria in the presence of a helper plasmid carrying the necessary transfer genes.

2. A plasmid cloning vehicle as in claim 1, wherein the cyanobacterial DNA sequence includes a cyanobacterial plasmid replication system.

3. A plasmid cloning vehicle as in claim 1, wherein the cyanobacterial DNA sequence is homologous to a region of genomic cyanobacterial DNA.

4. A plasmid cloning vehicle as in claim 3, wherein the cyanobacterial DNA sequence includes a restriction site and the homologous region extends at least 500 base pairs in each direction from the restriction site.

5. A plasmid cloning vehicle as in claim 2, wherein the cyanobacterial plasmid is pUH24 or pUH25.

6. A plasmid cloning vehicle as in claim 1, wherein the origin of transfer and the conjugative replication system are derived from IncW or IncP plasmids.

7. A plasmid cloning vehicle as in claim 1, further including at least one marker allowing for selection of cyanobacterial transformants.

8. A plasmid cloning vehicle as in claim 1, having a unique insertion site in a marker.

9. A system for cloning foreign DNA fragments in cyanobacteria, said system comprising a plasmid cloning vehicle and a helper plasmid, wherein said plasmid cloning vehicle includes a replication system recognized by cyanobacteria and a replication system and origin of transfer derived from a conjugative plasmid, and said helper plasmid carries the transfer genes necessary for mobilization of the plasmid cloning vehicle, whereby the plasmid cloning vehicle may be conjugatively transferred from Gram-negative bacteria to strains of cyanobacteria in the presence of the helper plasmid.

10. A method for transferring a DNA fragment from Gram-negative bacteria to cyanobacteria, said method comprising:
inserting said DNA fragment into a plasmid cloning vehicle including a selectable marker and having (1) a cyanobacterial replication system and (2) a replication system and origin of transfer derived from a conjugative plasmid;
mobilizing said plasmid cloning vehicle in the Gram-negative bacteria in the presence of a helper plasmid so that conjugal transfer is effected to a desired cyanobacterial recipient host; and
selecting recipients based on said selective marker present on the cloning vehicle.

| | DOCUMENTS CONSIDERED TO BE RELEVANT | | EP 85102962.9 |
|---|---|---|---|

| Category | Citation of document with indication. where appropriate, of relevant passages | Relevant to claim | CLASSIFICATION OF THE APPLICATION (Int. Cl 4) |
|---|---|---|---|
| D,A | PROCEEDINGS OF THE NATIONAL ACADEMY OF SCIENCES OF THE UNITED STATES OF AMERICA, vol. 77, no. 3, March 1980 (Baltimore, USA)<br><br>C.A.M.J.J. VAN DEN HONDEL et al. "Introduction of transposon Tn 901 into a plasmid of Anacystis nidulans: Preparation for cloning in cyanobacteria" pages 1570-1574<br><br>* Totality * | 1 | C 12 N 15/00 |
| D,A | MOLECULAR & GENERAL GENETICS, vol. 184, 1981, (Springer International; Berlin, Heidelberg, New York)<br><br>C.J. KUHLEMEIER et al. "Vectors for Cloning in Cyanobacteria: Construction and Characterization of Two Recombinant Plasmids Capable of Transformation to Escherichia coli K12 and Anacystis nidulans R2" pages 249-254<br><br>* Totality * | 1 | |

TECHNICAL FIELDS
SEARCHED (Int. Cl 4)

C 12 N

The present search report has been drawn up for all claims

| Place of search | Date of completion of the search | Examiner |
|---|---|---|
| VIENNA | 02-07-1985 | WOLF |